(19) Europäisches Patentamt
European Patent Office
Office européen des brevets

(11) **EP 4 111 855 A1**

(12) **EUROPEAN PATENT APPLICATION**

(43) Date of publication:
**04.01.2023 Bulletin 2023/01**

(51) International Patent Classification (IPC):
***A01H 1/04*** *(2006.01)*

(21) Application number: **21182738.1**

(52) Cooperative Patent Classification (CPC):
**A01H 1/045**

(22) Date of filing: **30.06.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2021 PT 2021117308**

(71) Applicants:
• **INIAV - Instituto Nacional de Invesigação Agrária E Veterinária, I.P.**
**2780-159 Oeiras (PT)**
• **Centro de Biotecnologia Agrícola E Agro-Alimentar**
**Do Alentejo - Cebal**
**7800-295 Beja (PT)**

(72) Inventors:
• **DE MATOS, FERNANDA PAULA DA SILVA SIMÕES**
**BELAS (PT)**
• **DA COSTA, ANA RITA PEREIRA**
**ELVAS (PT)**
• **DE ALMEIDA, ANA SOFIA VIEIRA DIAS**
**LISBOA (PT)**
• **SERRA, OCTÁVIO MANUEL RIBEIRO**
**BRAGA (PT)**
• **PINHEIRO, NUNO MANUEL BARROSO**
**CAMPO MAIOR (PT)**
• **COUTINHO, JOSÉ NORBERTO PRATES**
**FRONTEIRA (PT)**
• **ALVES, SÍLVIA CARNEIRO**
**LISBOA (PT)**
• **DE MATOS, JOSÉ ANTÓNIO DOS SANTOS PEREIRA**
**BELAS (PT)**
• **MAÇÃS, BENVINDO MARTINS**
**ELVAS (PT)**
• **CAMPOS, PAULA SCOTTI LORENZINI BORGES**
**LISBOA (PT)**
• **SEMEDO, JOSÉ MANUEL FERREIRA NOBRE**
**AMADORA (PT)**

(74) Representative: **Alves Moreira, Pedro**
**RCF Protecting Innovation, S.A.**
**Rua Dom Francisco Manuel de Melo, 15, 3°**
**1070-085 Lisbon (PT)**

(54) **SNP BASED PANEL FOR MEDITERRANEAN WHEAT PLANT SELECTION AND BREEDING**

(57) The present invention relates to a set of 38 SNP markers for genotyping 38 SNP sites in the wheat genome associated with specific agronomic traits of interest for wheat cropping in the Mediterranean area. Further provided is a method to detect favourable wheat trait genetic loci for marker assisted selection (MAS) to select parents for wheat breeding under Mediterranean climate. The invention also provides a kit comprising the set of 38 SNP markers.

EP 4 111 855 A1

**Description**

**FIELD OF THE INVENTION**

The present invention relates to the field of biotechnology, in particular to a set of single nucleotide polymorphism (SNP) for marker assisted selection of wheat plants.

**BACKGROUND OF THE INVENTION**

Wheat is the third most-important cereal crop in the world. To meet future market demands, some of the most important breeding objectives include increasing total yields and the rate at which wheat breeding programs adapt to new and changing environments, each time more frequent, mainly caused by climatic reasons. In crop breeding, the interest of predicting the performance of candidate cultivars in the field has increased due to recent advances in molecular breeding technologies. However, the complexity of the wheat genome presents some challenges for applying new technologies in molecular marker identification generating large numbers of molecular markers (SNPs) for use in genetic analyses such as genomics-assisted breeding in a range of plant species. Genotyping of crops allows breeders to conduct genomic selection (GS) on germplasm of interest using all available markers (phenotypic and genotypic) for a population as predictors of a breeding value. GS integrates marker data from a training population with phenotypic and pedigree data collected on the same population to generate a prediction model. The model outputs genomic estimated breeding values (GEBVs) for all genotyped individuals within a breeding population. GEBV is used as a predictor of how well a plant will perform as a parent for crossing and generation advance in a breeding pipeline, based on the similarity of its genomic profile to other plants in the training population that are known to have performed well in the target environment(s). The advantage of genomics-assisted breeding is that genotypic data obtained from a seed or seedling can be used to predict the phenotypic performance of mature individuals without the need for extensive phenotypic evaluation over years and environments. Genomic selection models have already proven to be advantageous but they are still quite laborious requiring the access to high throughput genotyping platforms and well trained and experienced personnel on bioinformatics and biostatistics to conduct prediction based on mathematical models. Therefore there is a need to develop methods allowing the use of shorter panels of molecular markers that can be used for germplasm screening in local breeding plant stations.

Although bread and durum wheat are important crops cultivated worldwide, wheat cropping is strongly dependent on climatic conditions. According to recent data, despite the increasing wheat plantings in 2021 in the United States of America and Canada, adverse climatic conditions decreased yield prospects for the main winter wheat crop and consequently wheat production is expected to be below average. Although in European Union the expansion of winter wheat planting is expected to lead to higher production yields, wheat production in the Southern Mediterranean countries is mostly dependent on weather conditions and water availability, being especially fragile under very high spring temperatures and long periods of land drought.

Grain yield (GY) improvement is one of the most challenging objectives in wheat breeding due to the complex genetic architecture and low heritability. Wheat production in the Southern European region is facing problems of long periods of groundwater decreasing, limiting irrigation frequency and causing the abandonment of wheat crop production by farmers. Although conventional breeding attempts to increase grain yield potential, advances are relatively slow as GY is a complex trait, strongly associated with spike number per unit area, kernel number per spike and thousand-kernel weight (TKW). However, some traits have higher heritabilities ($h^2$) than GY, although also affecting it, such as grain shape, spike architecture, plant height (PH) or flag leaf related traits, impacting in processes like photosynthetic intensity, grain filling and dry matter translocation. The above mentioned traits are easier to select in small plots at the early stages of breeding programs.

Most improvements in grain yield, including in the Alentejo region, were mainly related to the use of dwarfing genes and 1BL.1RS translocation lines to increase kernels per square meter, biomass and harvest index and reduced pH. Nowadays, after this renewed gene fixation, new strategies for variation must be envisaged. One of these strategies consists of the understanding of detailed genetic structure combined with marker-assisted selection (MAS). MAS is becoming a routine technique to overcome yield bottleneck of conventional breeding in the improvement of wheat yield potential. However, MAS application depends on the number of trait genes strongly associated with molecular markers.

Although there are many reports on quantitative trait loci (QTL) mapping and genome-wide association study (GWAS) on wheat yield and related trait loci, only a few have been applied in selection of wheat in breeding programs for the Mediterranean area, those being mainly for durum wheat (see for example: Pascual, L., Ruiz, M., López-Fernández, M. et al. Genomic analysis of Spanish wheat landraces reveals their variability and potential for breeding. BMC Genomics 21, 122 (2020).; Soriano, J.M.; Sansaloni, C.; Ammar, K.; Royo, C. Labelling Selective Sweeps Used in Durum Wheat Breeding from a Diverse and Structured Panel of Landraces and Cultivars. Biology 2021, 10, 258.; Mérida-García R, Liu G, He S, Gonzalez-Dugo V, Dorado G, Gálvez S, et al. (2019) Genetic dissection of agronomic and quality traits based on association mapping and genomic selection approaches in durum wheat grown in Southern Spain. PLoS ONE 14(2): e0211718.; Mangini G, Gadaleta A, Colasuonno P, Marcotuli I, Signorile AM, Simeone R, et al. (2018) Genetic dissection of the relationships between grain yield components by genome-wide association mapping in a collection of tetraploid wheats. PLoS ONE 13(1): e0190162.).

Several wheat SNP arrays for genetic studies of yield, quality, disease resistance and stress tolerance were developed for genotyping. See Jin H, Wen WE, Liu JD, Zhai SN, Zhang Y, Yan J, Liu ZY, Xia XC, He ZH. Genomewide QTL mapping for wheat processing quality parameters in a Gaocheng 8901/ Zhoumai 16 recombinant inbred line population. Front Plant Sci. 2016;7:1032.; Liu JD, He ZH, Rasheed A, Wen WE, Yan J, Zhang PZ, Wan YX, Zhang Y, Xie CJ, Xia XC. Genome-wide association mapping of black point reaction in common wheat (Triticum aestivum L.). BMC Plant Biol. 2017;17:220.; Sun CW, Zhang FY, Yan XF, Zhang XF, Dong ZD, Cui DQ, Chen F. Genomewide association study for 13 agronomic traits reveals distribution of superior alleles in bread wheat from the yellow and Huai Valley of China. Plant Biotechnol J. 2017;15:953-69.; Valluru R, Reynolds MP, Davies WJ, Sukumaran S. Phenotypic and genomewide association analysis of spike ethylene in diverse wheat genotypes under heat stress. New Phytol. 2017;214:271-83.

Wheat 50K and 15K SNP arrays are available for selecting important traits in wheat breeding programs and include SNP markers derived from the 35K, 90 K and 660 K wheat SNP arrays. For all SNP arrays, protocols are standardized and the data generated between different laboratories is comparable. In theory, the position of the SNPs on the chip is unique in the chromosome or genome, thus largely avoiding the typing problems caused by the multi-copy gene SNP. These wheat SNP chips have been used for wheat population structure, genetic variation, selection evolution and genome-wide association analysis.

SNP markers are now the main tool for genetic studies and breeding of crop species. Analysis of GWAS data based on linkage disequilibrium (LD) provides a much higher resolution capacity to reveal the genetic pathway of complex traits. GWAS uses directly all the available germplasm bypassing the need of developing mapping segregation populations and allowing only one phenotyping cycle for multiple traits. The genetic variance of traits in crop species may be caused by a single SNP, but is more often attributed to several SNPs within a haplotype block and therefore, SNP-GWAS is being commonly applied in genetic studies of crop species.

In Mediterranean-climate regions, temperate cereals like wheat are usually exposed to a severe water deficit during the grain filling period. This water deficit leads to reductions in canopy photosynthesis and lower levels of assimilate transfer to the grain, leading to low kernel weights and GY. Analysis of candidate genes and genome-wide association studies (GWAS) have been performed in wheat to identify SNP markers controlling traits such as dehydration tolerance (Lorenz AJ, Hamblin MT, Jannink JL. Performance of single nucleotide polymorphisms versus haplotypes for genome-wide association analysis in barley. PLoS One. 2010;5:e14079.; Garg, B.; Lata, C.; Prasad, M. A study of the role of gene TaMYB2 and an associated SNP in dehydration tolerance in common wheat. Mol. Biol. Rep. 2012, 39, 10865-10871.), grain yield (Wang, S.-X.; Zhu, Y.-L.; Zhang, D.-X.; Shao, H.; Liu, P.; Hu, J.-B.; Zhang, H.; Zhang, H.-P.; Chang, C.; Lu, J.; et al. Genome-wide association study for grain yield and related traits in elite wheat varieties and advanced lines using SNP markers. PLoS ONE 2017, 12, e0188662.), leaf traits (Huang, S.; Sun, L.; Hu, X.; Wang, Y.; Zhang, Y.; Nevo, E.; Peng, J.; Sun, D. Associations of canopy leaf traits with SNP markers in durum wheat (Triticum turgidum L. durum (Desf.)). PLoS ONE 2018, 13, e0206226. (Huang, S.; Sun, L.; Hu, X.; Wang, Y.; Zhang, Y.; Nevo, E.; Peng, J.; Sun, D. Associations of canopy leaf traits with SNP markers in durum wheat (Triticum turgidum L. durum (Desf.)). PLoS ONE 2018, 13, e0206226.) and others (Guerra, F.P.; Yáñez, A.; Matus, I.; del Pozo, A. Genome-Wide Association of Stem Carbohydrate Accumulation and Remobilization during Grain Growth in Bread Wheat (Triticum aestivum L.) in Mediterranean Environments. Plants 2021, 10, 539.).

Documents disclosing single nucleotide polymorphisms and methods to detect them in wheat and other crops are known in the art such as CN105256044A that discloses a wheat molecular barcode based on a set of 43 SNPs for identification and discrimination of wheat varieties or CN107794308A that reveals specific SNP for identifying the traits of wheat grains, aiming mostly Asian cultivated wheat.

In view of the above, there is a need to identify stable markers to assist wheat breeding for the Mediterranean area. Moreover, there is a need for identification of a reasonable number of markers associated to important agronomic traits for climate resilience and grain production that can easily and immediately be tested in local breeding without a big investment in high throughput equipment.

## SUMMARY OF THE INVENTION

Surprisingly, the aforementioned problem was solved by the present invention.

The present invention provides a set of 38 SNP markers for genotyping 38 SNP sites in the wheat genome associated with specific agronomic traits of interest for wheat cropping in the Mediterranean area, the 38 SNP markers being AX-94620918, AX-94417303, AX-95199577, AX-94632981, AX-95192948, AX-94788624, AX-94527824, AX-94727639, AX-94498228, AX-94690456, AX-94386458, AX-94434561, AX-95227398, AX-94943234, AX-94465615, AX-94464372, AX-95161376, AX-94786426, AX-94600322, AX-94845724, AX-95224829, AX-94740863, AX-95140911, AX-94500018, AX-94478188, AX-94750708, AX-94438290, AX-95126960, AX-94558126, AX-95120137, AX-95009987, AX-94976788, AX-94418123, AX-94619314, AX-94659431, AX-95224463, AX-94418429 and AX-94426996.

In a preferred embodiment, the specific traits of interest are thousand-grain-weight, heading days, hectolitre mass, maturity days, plant height, protein content and grain yield in kg/ha.

The present invention also provides a method to detect favourable wheat trait genetic loci for marker assisted selection (MAS) to select parents for wheat breeding under Mediterranean climate, the method comprising the steps of: a) detecting the set of 38 SNP genotypes aforementioned in the plant DNA to be tested; b) comparing the genotypes of all 38 SNP loci of the tested germplasm.

The invention further provides a kit comprising a primer combination for wheat plant SNP genotyping of the above 38 markers associated with agronomic traits of interest for wheat breeding under the Mediterranean area and climate.

The set of 38 SNP markers is used in combination with PCR based methods for detecting polymorphism.

The set of 38 SNP markers SNP markers is used in combination with PCR based methods for genotyping SNP loci in the wheat genome.

The set of 38 SNP markers is user for detecting favourable wheat trait genetic loci to assist genomic selection of wheat seedlings from wheat breeding programs under Mediterranean climate.

## BRIEF DESCRIPTION OF THE DRAWINGS

Figure 1 shows the wheat Single Nucleotide Polymorphism (SNP) screening process scheme.

Figure 2 represents a Manhattan plot summarizing the genome-wide association study (GWAS) results for one of the traits [Yield (kg/ha)] using a mixed linear model (MLM). This graph shows the physical position (bp) of each tested SNP in wheat's *(T aestivum)* reference genome (per chromosome) on the X-axis and -log(10) P-values from the MLM on the Y-axis.

Figure 3 shows selected trait associated marker analysis for a subsample of bread wheat (117 lines or varieties).

Figure 4 shows probe sequences of each marker tested in Figure 3.

Figure 5 shows the tree diagram discriminating all wheat tested.

## DETAILED DESCRIPTION OF THE INVENTION

The object of the present invention is to provide a set of 38 SNP for genotyping 38 SNP sites in the wheat genome associated with specific agronomic traits of interest for wheat cropping and breeding in the Mediterranean area, the 38 SNP sites being AX-94620918, AX-94417303, AX-95199577, AX-94632981, AX-95192948, AX-94788624, AX-94527824, AX-94727639, AX-94498228, AX-94690456, AX-94386458, AX-94434561, AX-95227398, AX-94943234, AX-94465615, AX-94464372, AX-95161376, AX-94786426, AX-94600322, AX-94845724, AX-95224829, AX-94740863, AX-95140911, AX-94500018, AX-94478188, AX-94750708, AX-94438290, AX-95126960, AX-94558126, AX-95120137, AX-95009987, AX-94976788, AX-94418123, AX-94619314, AX-94659431, AX-95224463, AX-94418429 and AX-94426996.

The 38 SNP sites are as follows:

| Marker | Chr | Position in the Chr (cM) |
| --- | --- | --- |
| AX-94620918 | 1B | 658714142 |
| AX-94417303 | 1B | 662154416 |
| AX-95199577 | 1D | 479052654 |
| AX-94632981 | 1B | 660454560 |
| AX-95192948 | UNKNOWN | 2130 |
| AX-94788624 | 6D | 356737499 |

(continued)

| Marker | Chr | Position in the Chr (cM) |
|---|---|---|
| AX-94527824 | 1D | 412082431 |
| AX-94727639 | 6B | 158201834 |
| AX-94498228 | 6B | 191527596 |
| AX-94690456 | UNKNOWN | 2473 |
| AX-94386458 | 1A | 3392719 |
| AX-94434561 | 5D | 398606941 |
| AX-95227398 | 5D | 398782794 |
| AX-94943234 | 1D | 28574855 |
| AX-94465615 | 1B | 40878671 |
| AX-94464372 | 4A | 709612601 |
| AX-95161376 | 1B | 20164825 |
| AX-94786426 | 6B | 146838012 |
| AX-94600322 | 1D | 9799741 |
| AX-94845724 | UNKNOWN | 1106 |
| AX-95224829 | 5B | 1579438 |
| AX-94740863 | 4A | 725802640 |
| AX-95140911 | 2D | 651606397 |
| AX-94500018 | UNKNOWN | 2359 |
| AX-94478188 | UNKNOWN | 1920 |
| AX-94750708 | 2D | 117840331 |
| AX-94438290 | 1B | 216098693 |
| AX-95126960 | 7A | 11087391 |
| AX-94558126 | 1B | 447164883 |
| AX-95120137 | 3D | 175407295 |
| AX-95009987 | 3D | 558724167 |
| AX-94976788 | 7A | 645028871 |
| AX-94418123 | 3D | 559185141 |
| AX-94619314 | 5A | 502372312 |
| AX-94659431 | UNKNOWN | 1928 |
| AX-95224463 | 5D | 400280006 |
| AX-94418429 | 5A | 509656247 |
| AX-94426996 | 5A | 25618238 |

In one embodiment, it is an object of the invention to provide a set of 38 SNP for genotyping 38 SNP sites in the wheat genome associated with specific traits of interest for wheat cropping using SNP-GWAS on a panel of wheat varieties from a breeding program for the Mediterranean climate. Preferably, the selected traits are thousand-grain-weight (also called 1000KW.g (PMG)), heading days, hectolitre mass in kg/hL, maturity days, plant height in cm, protein content in DW and grain yield in kg/ha.

It is also an object of the present invention to provide a method to detect favourable wheat trait genetic loci for marker assisted selection (MAS) to select parents for wheat breeding under Mediterranean climate, the method comprising the steps of:

a) detecting the 38 SNP genotypes in the plant DNA to be tested;
b) comparing the genotypes of all 38 SNP loci of the tested germplasm.

In one embodiment, the set of 38 SNP in combination with PCR based methods detects polymorphism or genotyping SNP loci in the wheat genome. The PCR based methods are selected from, for example, RT-PCR, High Resolution Melting (HRM) and the like.

In another embodiment, the set of 38 SNP detects favourable wheat trait genetic loci to assist genomic selection of wheat seedlings from wheat breeding programs under Mediterranean climate.

It is an additional object of the present invention to provide a kit comprising a primer combination for wheat plant SNP genotyping of 38 markers associated with agronomic traits of interest for wheat breeding under the Mediterranean area and climate.

Therefore, the aims of the present invention are summarized as follows:

1) identifying stable wheat loci associated to specific agronomic traits using SNP-GWAS on a panel of wheat varieties.
2) detecting favourable wheat trait genetic loci for marker assisted selection (MAS) to select parents for wheat breeding under Mediterranean climate.
3) detecting favourable wheat trait genetic loci set of markers to assist genomic selection of wheat seedlings from wheat breeding programs under Mediterranean climate.
4) producing a kit comprising a primer combination for wheat plant SNP genotyping of markers associated to agronomic traits important for wheat breeding under the Mediterranean area.

Genotyping means the detection of a nucleotide base polymorphism in the wheat genome in a specific SNP site. Preferably, the nucleotide base polymorphism is associated with agronomic traits, e. g. grain yield (GY) or others favourable to wheat cropping in a panel of wheat varieties (Table 1) under Mediterranean climate (high temperature and drought resilience).

Single Nucleotide Polymorphism associated to the traits of interest were merged in a subset panel for easy and fast genotyping of wheat plants and uses on activities of wheat breeding and marker assisted selection under Mediterranean climate.

Surprisingly, the present invention shows that a small subset of 38 SNP sites is enough to improve the efficiency of selection of wheat germoplasm on wheat breeding under Mediterranean climate. This subset has proven that it is useful to identify and/or differentiate the varieties shown in Table 1.

EXAMPLES

Hereinafter, the present invention is described in more detail and specifically with reference to the examples, which are not intended to be limitative.

The experimental methods described in the examples are all conventional methods unless otherwise specified. The materials, reagents and the like used in the following examples are commercially available unless otherwise specified. In the quantitative experiments of the examples, three replicate experiments were set and the results were averaged.

**Example 1. Discovery of specific SNPs and design of specific primer sets**

Screening of SNP loci

The wheat used in this example consisted of 117 varieties of wheat as shown in Table 1 below. Wheat germoplasm, including local varieties and modern cultivars, were widely planted in wheat cultivation areas in Alentejo, Portugal, since 1940s. These materials carry important agronomic trait genes, drought tolerance genes, high quality, large grain, large ear and other favourable agronomic traits. Some materials have been used as backbone parents to produce new wheat varieties. The set of wheat from table 1 was used to screen SNPs. All the wheat lines or varieties are from "Instituto Nacional de Investigação Agrária e Veterinária, I. P. (INIAV)" - "Estação Nacional de Melhoramento de Plantas - ENMP" at Elvas, Portugal, and are agronomically cultivated and characterized under the Mediterranean climate, under drought and high spring/summer temperatures.

Table 1. List of 117 wheat varieties/advanced lines

| ID Breed | Species | Wheat line or variety from INIAV germplasm breeding panel |
|---|---|---|
| 22 | *Ta* | PAIVA |
| 26 | *Ta* | RESTAURAÇÃO |
| 102 | *Ta* | PIRANA |
| 127 | *Ta* | BOLONHA |
| 660 | *Ta* | ARDITO |
| 897 | *Ta* | GENTIL ROSSO |
| 927 | *Ta* | MOCHO DE ESPIGA QUADRADA |
| 940 | *Ta* | TEMPORÃO DE CORUCHE |
| 1339 | *Ta* | MOCHO DE ESPIGA BRANCA |
| 1357 | *Ta* | RIBEIRO |
| 1998 | *Ta* | TE 1518 |
| 2004 | *Ta* | TE 1414 |
| 2049 | *Ta* | AMAZONAS |
| 2065 | *Ta* | CAIA |
| 2109 | *Ta* | SUNCO/2*PASTOR |
| 2210 | *Ta* | NABÃO |
| 2650 | *Ta* | PYN"S"/BOW"S" |
| 3457 | *Ta* | VPRT2 x VPRT3 |
| 3484 | *Ta* | EUFRATES |
| 3485 | *Ta* | MONDEGO |
| 3486 | *Ta* | JORDÃO |
| 3487 | *Ta* | SEVER |
| 3511 | *Ta* | CÔA |
| 3538 | *Ta* | PASTOR//MUNIA/ALTAR |
| 3759 | *Ta* | ATTILA/3/3*BCN//TOBA97 |
| 3942 | *Ta* | DEGEBE |
| 4028 | *Ta* | (TJB368.251/BUC x TROCADERO) |
| 4112 | *Ta* | ASP"S"/BLT"S"//HAHN"S"*2/PRL"S " |
| 4281 | *Ta* | QG 78.5//2*INQALAB91*2/TUKURU |
| 4455 | *Ta* | ALMANSOR |
| 4458 | *Ta* | ROXO |
| 4527 | *Ta* | CHAPIO/3/BORL95/2*EXCALIBUR//E XCALIBUR |
| 5022 | *Ta* | NZT/BEZ1//ALD/4/NAD//TMP/CI!"$ =&/3/EMU |
| 5032 | *Ta* | WAXWING*2/4/SNI/TRAP#1/3/KAUZ* 2/TRAP//KAUZ |
| 5391 | *Ta* | INGENIO |
| 5403 | *Ta* | ADÁGIO |
| 5428 | *Ta* | KA/NAC//TRCH/3/VORB |
| 5446 | *Ta* | BABAX/LR42/ /BABAX*2/4/SNI/TRAP 31/3/KAUZ*27TRAP/ /KAUZ75/WHEAR 7SOKOLL |

(continued)

| ID Breed | Species | Wheat line or variety from INIAV germplasm breeding panel |
|---|---|---|
| 5464 | *Ta* | ND643/2*WBLL1/4/CHIBIA/ /PRLI/C M6553173/SKAUZ/BAV92/5/BECARD |
| 5472 | *Ta* | BAVIS//ATTILA*2/PBW65 |
| 5505 | *Ta* | KACHU*2//WHEAR/SOKOLL |
| 5512 | *Ta* | GALERA |
| 5614 | *Ta* | ENEBRO |
| 5615 | *Ta* | AVELINO |
| 5616 | *Ta* | MONTE CARLO |
| 5620 | *Ta* | ANTEQUERA |
| 5953 | *Ta* | (FLORIDA302 X TROCADERO) X (PRL/SARA/ /TSI/VEE#5...) |
| 5997 | *Ta* | (CORDIAL X KI/PMFN/3//77C/CNO67... .) X TE0206 |
| 6110 | *Ta* | KATUNGA X (CENTAURO/VEJA) |
| 6380 | *Ta* | (TAST/SPRW//ZAR/5/YUANDONG3/4/ PPBB-68/CHRC/3/PYN/ /TAM101/AMI... |
| 6425 | *Ta* | ACCROC |
| 6461 | *Ta* | MH12-32 |
| 6592 | *Ta* | MH12-24 |
| 6684 | *Ta* | KWS W204 |
| 6704 | *Ta* | KWS W227 |
| 6719 | *Ta* | SY10110 |
| 7129 | *Ta* | CHIBIA//PRLII/CM65531/3/5KAUZ/ BAV92/4/... |
| 7514 | *Ta* | KAUZ"S"/SERI/3/TEVEE"S"//CROW/ |
| | | VEE"S" |
| 8020 | *Ta* | SOKOLL//PU894.15.1.12/WBLL1 |
| 8173 | *Ta* | VORB74/D67.2/PARANA 66.270//AE.SQUARROSA (3209/37CUNNIGHAM/5/D67.2.. |
| 8173 | *Ta* | VORB/4/D67.2/PARANA66.270//AE. SQUARROSA (320) /3/... |
| 8310 | *Ta* | SUR.388-150 |
| 8312 | *Ta* | FILON |
| 8315 | *Ta* | MAUPASSANT |
| 8325 | *Ta* | (GOLIA X CEZANNE) X (IDEAL X TE8902) |
| 8326 | *Ta* | (KENNEDY X ROXO) |
| 8349 | *Ta* | AMUR/3/KINGBIRD#1/ /INQALAB91*2 /TUKURU/4/AMUR |
| 8352 | *Ta* | BABAX/LR$"/ /BABAX*2/KUKURU/4/T X96V2427 |
| 8353 | *Ta* | BABAX/LR42/ /BABAX*2/3/VIVITS/4 /AGRI/NAC//ATTILA |
| 8364 | *Ta* | NEOSHO/KS980508-1~1 |
| 8364 | *Ta* | NEOSHO/KS980508-1~1 |
| 8371 | *Ta* | DANPHE#1*2/SOLALA |
| 8374 | *Ta* | GRK79//INQALAB91*2/TUKURU |
| 8403 | *Ta* | PASTOR/MILAN/7/ZCL73/PGFN//CNO 67//SERI/5/UA.2837/... |
| 8411 | *Ta* | PFAU/SERI.18//AMAD/3/WAXWING*2 |

(continued)

| ID Breed | Species | Wheat line or variety from INIAV germplasm breeding panel |
|---|---|---|
| | | /4/BECARD |
| 8422 | *Ta* | TACUPETO F200176/CNDO/R143//ENTE/MEXI_2 /3/AEGILOPS SQUARROSA (TAUS)/ 4/WEAVER/... . . |
| 8428 | *Ta* | SAUAL74/CROC_1/AE.SQUARROSA 82059//KAUZ/3/ATTILA/5/SAUAL/8 /TACUPETO F2001/6/.... |
| 8438 | *Ta* | KACHU#1/3/C80.1/3*BATAVIA//2*W BLL/4/KACHU/8/TACUPETO F2001/6/CNDO/R143// ENTE/... |
| 8485 | *Ta* | (ARPIGE x ARCHAMP) x ARPIGE |
| 8661 | *Ta* | PFAU7SERI.18/ /AMAD/3/WAXWING*2 /47TECU#1 |
| 8670 | *Ta* | BAV92/SERI |
| 8886 | *Ta* | KACHU*2/BECARD |
| 8975 | *Ta* | (GOLIA X CEZANNE) X (IDEAL X TE8902) |
| 8975 | *Ta* | (GOLIA X CEZANNE) X (IDEAL X TE8902) |
| 9028 | *Ta* | (GOELENT x BOLONHA) x ARDILA |
| 9028 | *Ta* | (GOELENT X BOLONHA) X ARDILA |
| 9029 | *Ta* | (GOELENT X BOLONHA) X ARDILA |
| 9048 | *Ta* | (KENNEDY x ROXO) |
| 9167 | *Ta* | (ORION X HEREWARD) |
| 9167 | *Ta* | (ORION x HEREWARD) |
| 9170 | *Ta* | (ORION X HEREWARD) |
| 9231 | *Ta* | (FILIN/MILAN) x (TNMU/PASTOR) |
| 9377 | *Ta* | KAUZ//ALTAR84/AOS/3/TNMU/MILAN /4/MILAN//PSN/BOW |
| 9396 | *Ta* | NOGAL |
| 9578 | *Ta* | KWSW22 0 |
| 9579 | *Ta* | INTENSIVNAYA/KUKUNA |
| 9581 | *Ta* | WAXBI*2/COPIO |
| 9582 | *Ta* | KWSW222 |
| 9583 | *Ta* | MACARENO |
| 9584 | *Ta* | AFICIÒN |
| 9585 | *Ta* | B2019_2 |
| 9586 | *Ta* | ELS12-61 |
| 9588 | *Ta* | ACORAZADO |
| 9589 | *Ta* | TE 2002 |
| 9590 | *Ta* | MUTUS*2/TECUE#1/3/KINGBIRB#1/ / INQALAB91*2/TUKURU |
| 9591 | *Ta* | BIZÃNCIO |
| 9592 | *Ta* | ELYSIM |
| 9593 | *Ta* | KWSW252 |
| 9595 | *Ta* | WHEAR/ /2*PRL/2*PASTOR/3/WAXBI/ |
| | | 4/COPIO |

(continued)

| ID Breed | Species | Wheat line or variety from INIAV germplasm breeding panel |
|---|---|---|
| 9596 | *Ta* | KWSW261 |
| 9597 | *Ta* | RW41269 (RGT Pembroke) |
| 9598 | *Ta* | TE 2003 |
| 9599 | *Ta* | SPN/NAC//ATTILA/3/SHARK/F4105W 2.1 |
| 9602 | *Ta* | CNDO/R143//ENTE/MEXI_2/3/AEILO PS SQUARROSA... |
| 9604 | *Ta* | CNDO/R148//ENTE/MEX_2/3/AE.SQU ARROSA... |
| 9607 | *Ta* | SERI*3//RL6010/4*YR/3/PASTOR/4 /BAV92/5/... |
| 9613 | *Ta* | (ATTILA/3/AGRI/NAC//MLT)XID#37 01/6/VPM/MOS83-11-4-8/ /PEW/7/... . . |
| *Ta* = *Triticum* aestivum | | |

1) DNA extraction

All the materials shown in Table 1 were DNA extracted from seeds using a DNA extraction kit (AnalytycJena, Germany) according to the manufacturer's instructions.

2) Genotype detection

Samples were quantified and when passing the quality test (1.8<OD260/280<2.0), normalized to a concentration of 23 ng/μL and distributed to 96 well PCR plates. SNP genotyping was performed as a service at Segalab (Portugal) using the Axiom™ Wheat Breeder's Genotyping Array.

Axiom summary files were converted to the desired fitPoly format using a custom python script. In detail, data was rearranged into columns "MarkerName", "SampleName", "X", "Y", "R" and "ratio" to accommodate the name of the SNP marker, the name of the sample, the A-allele intensity, the B-allele intensity, the sum of A and B intensities, and the ratio of intensity calculated as A/(B+A), respectively.

The resulting converted data files were then imported to R [38] in order to assign genotypes to all samples using R package "fitPoly" [39]. For each marker, function "saveMarkerModels" was employed, which fitted multiple mixture models and selected the best fitting model based on the Bayesian Information Criterion (BIC), which takes into account the Log-Likelihood and the number of fitted parameters of the models.

Genotypes were only accepted if they met several thresholds, namely p.threshold > 0.99 (default), call.threshold > 0.6 (default) and peak.threshold (default 0.85, was changed to 1.0 to allow for monomorphic markers). Genotype scores attributed by FitPoly (0 to 6) were diploidized for further use. In detail, numerical genotypes from FitPoly corresponding to the 6 possible allelic combinations (0 to 6) were converted to diploid equivalence, as follows: 0's were converted to homozygous genotype for one parent, codes 1-5 were converted to heterozygous genotype and 6's were converted to homozygous genotype for the other parent. The result of this diploidization was then converted into variant call format (VCF) in order to be used downstream for genotype-phenotype association studies.

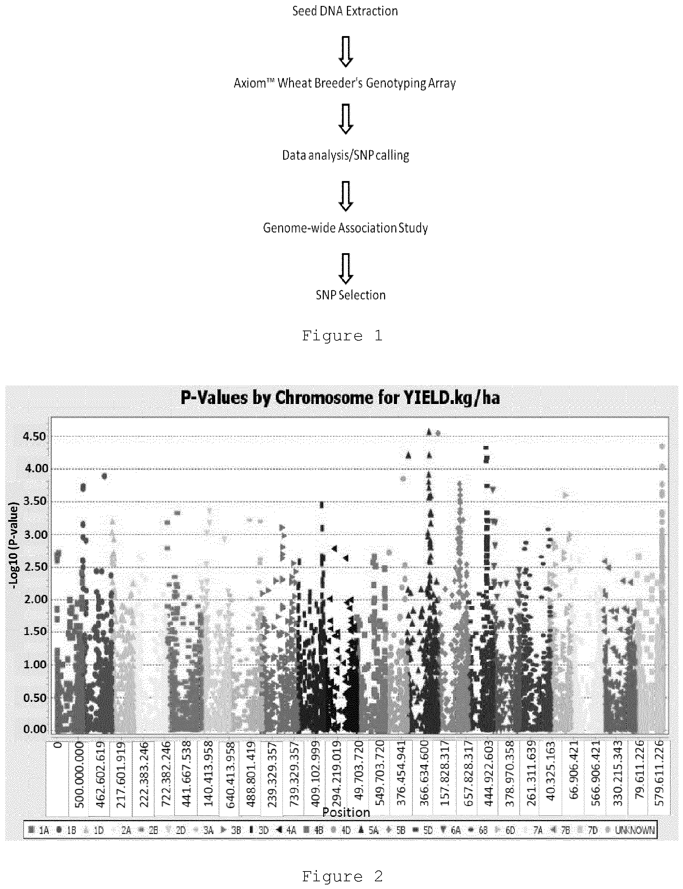

The SNP screening process based on 117 wheat varieties was according the scheme shown in Figure 1.

3) Genome-wide association study for SNP panel selection

For identifying which SNP variants are associated with the specific traits in analysis, a standard Java-based software named TASSEL (Trait Analysis by aSSociation, Evolution and Linkage) was employed. TASSEL software for genome wide association analysis (GWAS) implements both general linear model (GLM) and mixed linear model (MLM) approaches for controlling population and family structure. In order to account for population structure, a Principal Component Analysis (PCA) was performed and the top five Principal Components (PCs) were used as covariates. Family structure was accounted for via a matrix of kinship coefficients (kinship matrix) that TASSEL calculates separately.

The results presented in Figure 2 were obtained via the Q + K method, which uses a mixed linear model (MLM) function that can be described as follows:

$$y = X\beta + Z\mu + e$$

where "y" is the vector of observed phenotypes; "β" is an unknown vector containing fixed effects including genetic marker and population structure (Q); "μ" is an unknown vector of random additive genetic effects from multiple lines; "X" and "Z" are the known design matrices; and "e" is the unobserved vector of random residuals.

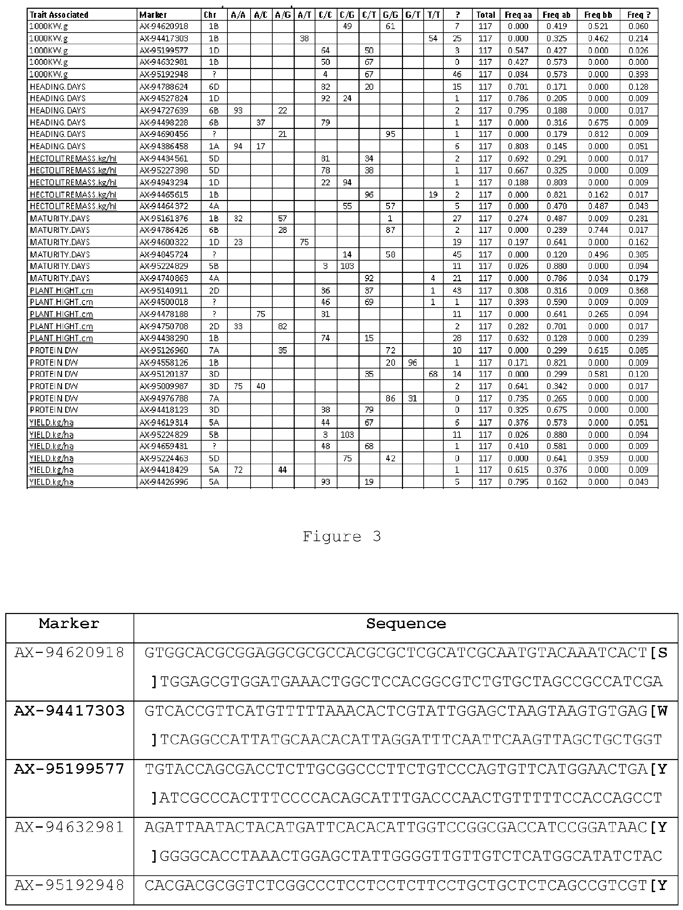

The analysis of genotyping of wheat lines from Table 1 with the 38 SNP sites panel has provided the results shown in Figure 3, thus making evidence of genotype frequencies.

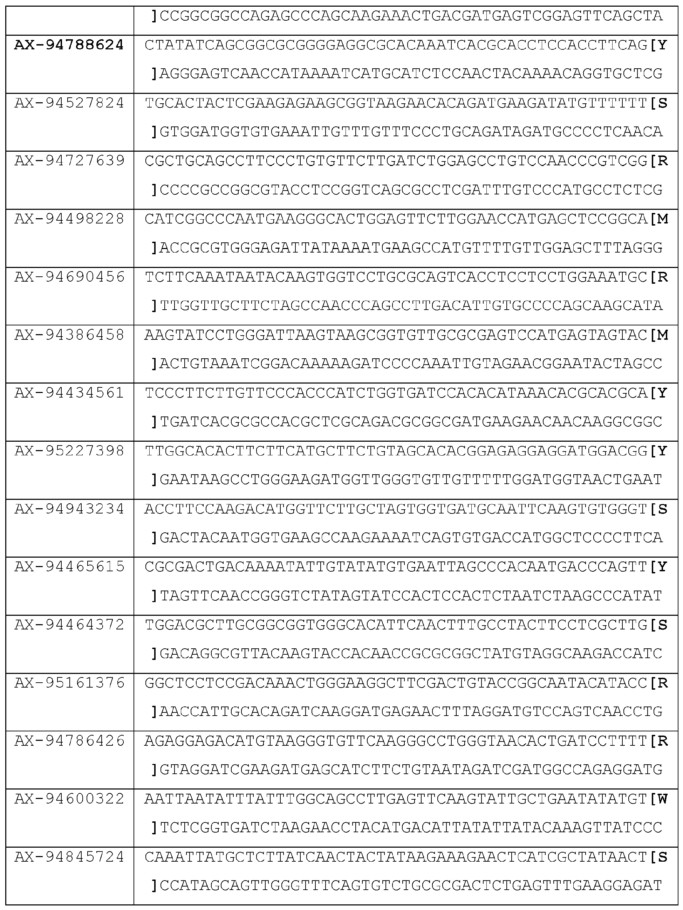

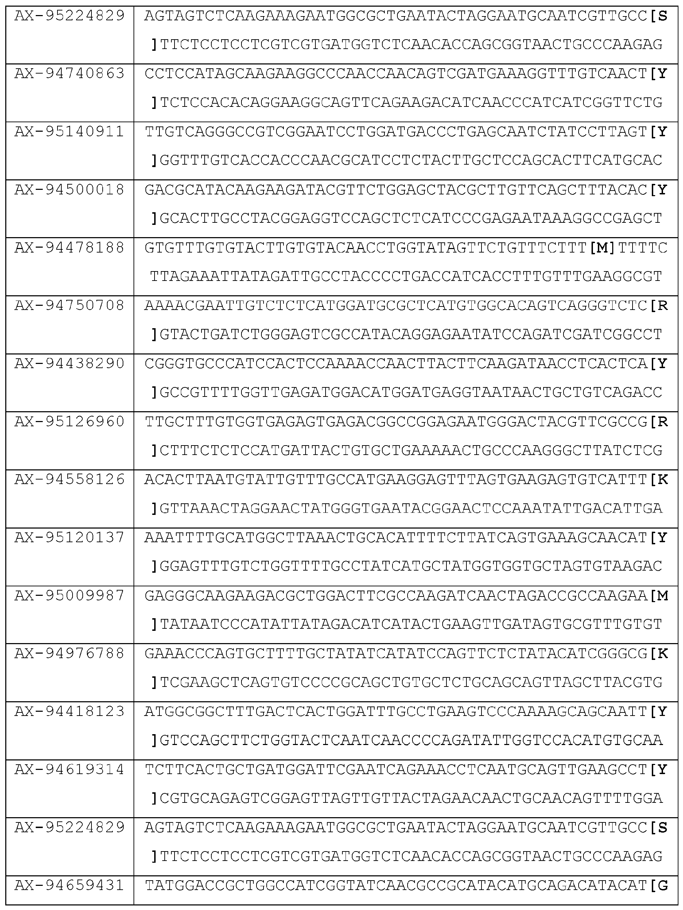

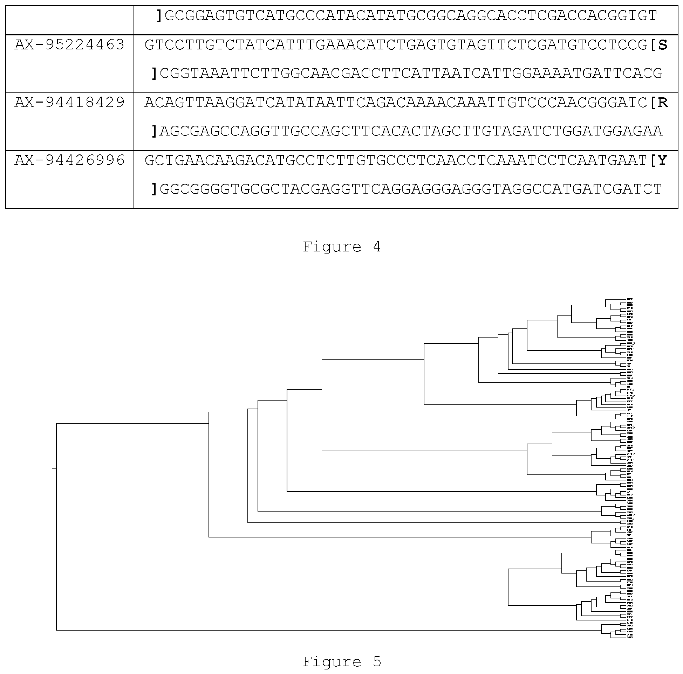

The sequence of each of the 38 SNP markers selected in this invention is shown in Figure 4.

**Example** 2. **38 SNP panel in the differentiation of wheat varieties**

Genotype data for the 38 selected SNPs across the 117 bread wheat varieties was loaded into TASSEL software. The matrix of genetic distances between samples was calculated within the software, following a modified euclidean distance model, where distances are calculated as 1 minus the probability that alleles drawn at random from two individuals at the same locus are the same, or 1-IBS (Identical by state). This distance matrix was then used by the software to create a Neighbor-joining tree, as shown in Figure 5.

The 38 SNP selected markers are able to discriminate/identify all the samples tested, and can therefore be used to identify a wheat variety/advanced line.

## Claims

1. A set of 38 SNP markers for genotyping 38 SNP sites in the wheat genome associated with specific agronomic traits of interest for wheat cropping in the Mediterranean area, the 38 SNP markers being AX-94620918, AX-94417303, AX-95199577, AX-94632981, AX-95192948, AX-94788624, AX-94527824, AX-94727639, AX-94498228, AX-94690456, AX-94386458, AX-94434561, AX-95227398, AX-94943234, AX-94465615, AX-94464372, AX-95161376, AX-94786426, AX-94600322, AX-94845724, AX-95224829, AX-94740863, AX-95140911, AX-94500018, AX-94478188, AX-94750708, AX-94438290, AX-95126960, AX-94558126, AX-95120137, AX-95009987, AX-94976788, AX-94418123, AX-94619314, AX-94659431, AX-95224463, AX-94418429 and AX-94426996.

2. The set of 38 SNP markers of claim 1 wherein the specific traits of interest are thousand-grain-weight, heading days, hectolitre mass, maturity days, plant height, protein content and grain yield in kg/ha.

3. A method to detect favourable wheat trait genetic loci for marker assisted selection (MAS) to select parents for wheat breeding under Mediterranean climate, the method comprising the steps of: a) detecting the set of 38 SNP genotypes of claim 1 in the plant DNA to be tested; b) comparing the genotypes of all 38 SNP loci of the tested germplasm.

4. Kit comprising a primer combination for wheat plant SNP genotyping of 38 markers according to claim 1 associated with agronomic traits of interest for wheat breeding under the Mediterranean area and climate.

5. Use of the set of 38 SNP markers of claim 1 in combination with PCR based methods for detecting polymorphism.

6. Use of the set of 38 SNP markers of claim 1 in combination with PCR based methods for genotyping SNP loci in the wheat genome.

7. Use of the set of 38 SNP markers of claim 1 for detecting favourable wheat trait genetic loci to assist genomic selection of wheat seedlings from wheat breeding programs under Mediterranean climate.

Seed DNA Extraction

⇓

Axiom™ Wheat Breeder's Genotyping Array

⇓

Data analysis/SNP calling

⇓

Genome-wide Association Study

⇓

SNP Selection

Figure 1

Figure 2

| Trait Associated | Marker | Chr | A/A | A/C | A/G | A/T | C/C | C/G | C/T | G/G | G/T | T/T | ? | Total | Freq aa | Freq ab | Freq bb | Freq ? |
|---|---|---|---|---|---|---|---|---|---|---|---|---|---|---|---|---|---|---|
| 1000KW.g | AX-94620918 | 1B | | | | | | 49 | | 61 | | | 7 | 117 | 0.000 | 0.419 | 0.521 | 0.060 |
| 1000KW.g | AX-94417303 | 1B | | | | 38 | | | | | | 54 | 25 | 117 | 0.000 | 0.325 | 0.462 | 0.214 |
| 1000KW.g | AX-95199577 | 1D | | | | | 64 | | 50 | | | | 3 | 117 | 0.547 | 0.427 | 0.000 | 0.026 |
| 1000KW.g | AX-94632981 | 1B | | | | | 50 | | 67 | | | | 0 | 117 | 0.427 | 0.573 | 0.000 | 0.000 |
| 1000KW.g | AX-95192948 | ? | | | | | 4 | | 67 | | | | 46 | 117 | 0.034 | 0.573 | 0.000 | 0.393 |
| HEADING.DAYS | AX-94788624 | 6D | | | | | 82 | | 20 | | | | 15 | 117 | 0.701 | 0.171 | 0.000 | 0.128 |
| HEADING.DAYS | AX-94527824 | 1D | | | | | 92 | 24 | | | | | 1 | 117 | 0.786 | 0.205 | 0.000 | 0.009 |
| HEADING.DAYS | AX-94727639 | 6B | 93 | | 22 | | | | | | | | 2 | 117 | 0.795 | 0.188 | 0.000 | 0.017 |
| HEADING.DAYS | AX-94498228 | 6B | | 37 | | | 79 | | | | | | 1 | 117 | 0.000 | 0.316 | 0.675 | 0.009 |
| HEADING.DAYS | AX-94690456 | ? | | | 21 | | | | | 95 | | | 1 | 117 | 0.000 | 0.179 | 0.812 | 0.009 |
| HEADING.DAYS | AX-94386458 | 1A | 94 | 17 | | | | | | | | | 6 | 117 | 0.803 | 0.145 | 0.000 | 0.051 |
| HECTOLITREMASS.kg/hl | AX-94434561 | 5D | | | | | 81 | | 34 | | | | 2 | 117 | 0.692 | 0.291 | 0.000 | 0.017 |
| HECTOLITREMASS.kg/hl | AX-95227398 | 5D | | | | | 78 | | 38 | | | | 1 | 117 | 0.667 | 0.325 | 0.000 | 0.009 |
| HECTOLITREMASS.kg/hl | AX-94943234 | 1D | | | | | 22 | 94 | | | | | 1 | 117 | 0.188 | 0.803 | 0.000 | 0.009 |
| HECTOLITREMASS.kg/hl | AX-94465615 | 1B | | | | | | | 96 | | | 19 | 2 | 117 | 0.000 | 0.821 | 0.162 | 0.017 |
| HECTOLITREMASS.kg/hl | AX-94464372 | 4A | | | | | | 55 | | 57 | | | 5 | 117 | 0.000 | 0.470 | 0.487 | 0.043 |
| MATURITY.DAYS | AX-95161376 | 1B | 32 | | 57 | | | | | 1 | | | 27 | 117 | 0.274 | 0.487 | 0.009 | 0.231 |
| MATURITY.DAYS | AX-94786426 | 6B | | | 28 | | | | | 87 | | | 2 | 117 | 0.000 | 0.239 | 0.744 | 0.017 |
| MATURITY.DAYS | AX-94600322 | 1D | 23 | | | 75 | | | | | | | 19 | 117 | 0.197 | 0.641 | 0.000 | 0.162 |
| MATURITY.DAYS | AX-94845724 | ? | | | | | | | 14 | 58 | | | 45 | 117 | 0.000 | 0.120 | 0.496 | 0.385 |
| MATURITY.DAYS | AX-95224829 | 5B | | | | | 3 | 103 | | | | | 11 | 117 | 0.026 | 0.880 | 0.000 | 0.094 |
| MATURITY.DAYS | AX-94740863 | 4A | | | | | | | 92 | | | 4 | 21 | 117 | 0.000 | 0.786 | 0.034 | 0.179 |
| PLANT.HIGHT.cm | AX-95140911 | 2D | | | | | 86 | | 87 | | | 1 | 43 | 117 | 0.308 | 0.316 | 0.009 | 0.368 |
| PLANT.HIGHT.cm | AX-94500018 | ? | | | | | 46 | | 69 | | | 1 | 1 | 117 | 0.393 | 0.590 | 0.009 | 0.009 |
| PLANT.HIGHT.cm | AX-94478188 | ? | | 75 | | | 31 | | | | | | 11 | 117 | 0.000 | 0.641 | 0.265 | 0.094 |
| PLANT.HIGHT.cm | AX-94750708 | 2D | 33 | | 82 | | | | | | | | 2 | 117 | 0.282 | 0.701 | 0.000 | 0.017 |
| PLANT.HIGHT.cm | AX-94438290 | 1B | | | | | 74 | | 15 | | | | 28 | 117 | 0.632 | 0.128 | 0.000 | 0.239 |
| PROTEIN.DW | AX-95126960 | 7A | | | 35 | | | | | 72 | | | 10 | 117 | 0.000 | 0.299 | 0.615 | 0.085 |
| PROTEIN.DW | AX-94558126 | 1B | | | | | | | 20 | | 96 | | 1 | 117 | 0.171 | 0.821 | 0.000 | 0.009 |
| PROTEIN.DW | AX-95120137 | 3D | | | | | | | 35 | | | 68 | 14 | 117 | 0.000 | 0.299 | 0.581 | 0.120 |
| PROTEIN.DW | AX-95009987 | 3D | 75 | 40 | | | | | | | | | 2 | 117 | 0.641 | 0.342 | 0.000 | 0.017 |
| PROTEIN.DW | AX-94976788 | 7A | | | | | | | | 86 | 31 | | 0 | 117 | 0.735 | 0.265 | 0.000 | 0.000 |
| PROTEIN.DW | AX-94418123 | 3D | | | | | 38 | | 79 | | | | 0 | 117 | 0.325 | 0.675 | 0.000 | 0.000 |
| YIELD.kg/ha | AX-94619314 | 5A | | | | | 44 | | 67 | | | | 6 | 117 | 0.376 | 0.573 | 0.000 | 0.051 |
| YIELD.kg/ha | AX-95224829 | 5B | | | | | 3 | 103 | | | | | 11 | 117 | 0.026 | 0.880 | 0.000 | 0.094 |
| YIELD.kg/ha | AX-94659431 | ? | | | | | 48 | | 68 | | | | 1 | 117 | 0.410 | 0.581 | 0.000 | 0.009 |
| YIELD.kg/ha | AX-95224463 | 5D | | | | | | 75 | | 42 | | | 0 | 117 | 0.000 | 0.641 | 0.359 | 0.000 |
| YIELD.kg/ha | AX-94418429 | 5A | 72 | | 44 | | | | | | | | 1 | 117 | 0.615 | 0.376 | 0.000 | 0.009 |
| YIELD.kg/ha | AX-94426996 | 5A | | | | | 93 | | 19 | | | | 5 | 117 | 0.795 | 0.162 | 0.000 | 0.043 |

Figure 3

| Marker | Sequence |
|---|---|
| AX-94620918 | GTGGCACGCGGAGGCGCGCCACGCGCTCGCATCGCAATGTACAAATCACT[S]TGGAGCGTGGATGAAACTGGCTCCACGGCGTCTGTGCTAGCCGCCATCGA |
| **AX-94417303** | GTCACCGTTCATGTTTTTAAACACTCGTATTGGAGCTAAGTAAGTGTGAG[W]TCAGGCCATTATGCAACACATTAGGATTTCAATTCAAGTTAGCTGCTGGT |
| **AX-95199577** | TGTACCAGCGACCTCTTGCGGCCCTTCTGTCCCAGTGTTCATGGAACTGA[Y]ATCGCCCACTTTCCCCACAGCATTTGACCCAACTGTTTTTCCACCAGCCT |
| AX-94632981 | AGATTAATACTACATGATTCACACATTGGTCCGGCGACCATCCGGATAAC[Y]GGGGCACCTAAACTGGAGCTATTGGGGTTGTTGTCTCATGGCATATCTAC |
| AX-95192948 | CACGACGCGGTCTCGGCCCTCCTCCTCTTCCTGCTGCTCTCAGCCGTCGT[Y |

| | |
|---|---|
| | ]CCGGCGGCCAGAGCCCAGCAAGAAACTGACGATGAGTCGGAGTTCAGCTA |
| AX-94788624 | CTATATCAGCGGCGCGGGGAGGCGCACAAATCACGCACCTCCACCTTCAG[Y ]AGGGAGTCAACCATAAAATCATGCATCTCCAACTACAAAACAGGTGCTCG |
| AX-94527824 | TGCACTACTCGAAGAGAAGCGGTAAGAACACAGATGAAGATATGTTTTTT[S ]GTGGATGGTGTGAAATTGTTTGTTTCCCTGCAGATAGATGCCCCTCAACA |
| AX-94727639 | CGCTGCAGCCTTCCCTGTGTTCTTGATCTGGAGCCTGTCCAACCCGTCGG[R ]CCCCGCCGGCGTACCTCCGGTCAGCGCCTCGATTTGTCCCATGCCTCTCG |
| AX-94498228 | CATCGGCCCAATGAAGGGCACTGGAGTTCTTGGAACCATGAGCTCCGGCA[M ]ACCGCGTGGGAGATTATAAAATGAAGCCATGTTTTGTTGGAGCTTTAGGG |
| AX-94690456 | TCTTCAAATAATACAAGTGGTCCTGCGCAGTCACCTCCTCCTGGAAATGC[R ]TTGGTTGCTTCTAGCCAACCCAGCCTTGACATTGTGCCCCAGCAAGCATA |
| AX-94386458 | AAGTATCCTGGGATTAAGTAAGCGGTGTTGCGCGAGTCCATGAGTAGTAC[M ]ACTGTAAATCGGACAAAAAGATCCCCAAATTGTAGAACGGAATACTAGCC |
| AX-94434561 | TCCCTTCTTGTTCCCACCCATCTGGTGATCCACACATAAACACGCACGCA[Y ]TGATCACGCGCCACGCTCGCAGACGCGGCGATGAAGAACAACAAGGCGGC |
| AX-95227398 | TTGGCACACTTCTTCATGCTTCTGTAGCACACGGAGAGGAGGATGGACGG[Y ]GAATAAGCCTGGGAAGATGGTTGGGTGTTGTTTTTGGATGGTAACTGAAT |
| AX-94943234 | ACCTTCCAAGACATGGTTCTTGCTAGTGGTGATGCAATTCAAGTGTGGGT[S ]GACTACAATGGTGAAGCCAAGAAAATCAGTGTGACCATGGCTCCCCTTCA |
| AX-94465615 | CGCGACTGACAAAATATTGTATATGTGAATTAGCCCACAATGACCCAGTT[Y ]TAGTTCAACCGGGTCTATAGTATCCACTCCACTCTAATCTAAGCCCATAT |
| AX-94464372 | TGGACGCTTGCGGCGGTGGGCACATTCAACTTTGCCTACTTCCTCGCTTG[S ]GACAGGCGTTACAAGTACCACAACCGCGCGGCTATGTAGGCAAGACCATC |
| AX-95161376 | GGCTCCTCCGACAAACTGGGAAGGCTTCGACTGTACCGGCAATACATACC[R ]AACCATTGCACAGATCAAGGATGAGAACTTTAGGATGTCCAGTCAACCTG |
| AX-94786426 | AGAGGAGACATGTAAGGGTGTTCAAGGGCCTGGGTAACACTGATCCTTTT[R ]GTAGGATCGAAGATGAGCATCTTCTGTAATAGATCGATGGCCAGAGGATG |
| AX-94600322 | AATTAATATTTATTTGGCAGCCTTGAGTTCAAGTATTGCTGAATATATGT[W ]TCTCGGTGATCTAAGAACCTACATGACATTATATTATACAAAGTTATCCC |
| AX-94845724 | CAAATTATGCTCTTATCAACTACTATAAGAAAGAACTCATCGCTATAACT[S ]CCATAGCAGTTGGGTTTCAGTGTCTGCGCGACTCTGAGTTTGAAGGAGAT |

| AX-95224829 | AGTAGTCTCAAGAAAGAATGGCGCTGAATACTAGGAATGCAATCGTTGCC[S ]TTCTCCTCCTCGTCGTGATGGTCTCAACACCAGCGGTAACTGCCCAAGAG |
|---|---|
| AX-94740863 | CCTCCATAGCAAGAAGGCCCAACCAACAGTCGATGAAAGGTTTGTCAACT[Y ]TCTCCACACAGGAAGGCAGTTCAGAAGACATCAACCCATCATCGGTTCTG |
| AX-95140911 | TTGTCAGGGCCGTCGGAATCCTGGATGACCCTGAGCAATCTATCCTTAGT[Y ]GGTTTGTCACCACCCAACGCATCCTCTACTTGCTCCAGCACTTCATGCAC |
| AX-94500018 | GACGCATACAAGAAGATACGTTCTGGAGCTACGCTTGTTCAGCTTTACAC[Y ]GCACTTGCCTACGGAGGTCCAGCTCTCATCCCGAGAATAAAGGCCGAGCT |
| AX-94478188 | GTGTTTGTGTACTTGTGTACAACCTGGTATAGTTCTGTTTCTTT[M]TTTTC TTAGAAATTATAGATTGCCTACCCCTGACCATCACCTTTGTTTGAAGGCGT |
| AX-94750708 | AAAACGAATTGTCTCTCATGGATGCGCTCATGTGGCACAGTCAGGGTCTC[R ]GTACTGATCTGGGAGTCGCCATACAGGAGAATATCCAGATCGATCGGCCT |
| AX-94438290 | CGGGTGCCCATCCACTCCAAAACCAACTTACTTCAAGATAACCTCACTCA[Y ]GCCGTTTTGGTTGAGATGGACATGGATGAGGTAATAACTGCTGTCAGACC |
| AX-95126960 | TTGCTTTGTGGTGAGAGTGAGACGGCCGGAGAATGGGACTACGTTCGCCG[R ]CTTTCTCTCCATGATTACTGTGCTGAAAAACTGCCCAAGGGCTTATCTCG |
| AX-94558126 | ACACTTAATGTATTGTTTGCCATGAAGGAGTTTAGTGAAGAGTGTCATTT[K ]GTTAAACTAGGAACTATGGGTGAATACGGAACTCCAAATATTGACATTGA |
| AX-95120137 | AAATTTTGCATGGCTTAAACTGCACATTTTCTTATCAGTGAAAGCAACAT[Y ]GGAGTTTGTCTGGTTTTGCCTATCATGCTATGGTGGTGCTAGTGTAAGAC |
| AX-95009987 | GAGGGCAAGAAGACGCTGGACTTCGCCAAGATCAACTAGACCGCCAAGAA[M ]TATAATCCCATATTATAGACATCATACTGAAGTTGATAGTGCGTTTGTGT |
| AX-94976788 | GAAACCCAGTGCTTTTGCTATATCATATCCAGTTCTCTATACATCGGGCG[K ]TCGAAGCTCAGTGTCCCCGCAGCTGTGCTCTGCAGCAGTTAGCTTACGTG |
| AX-94418123 | ATGGCGGCTTTGACTCACTGGATTTGCCTGAAGTCCCAAAAGCAGCAATT[Y ]GTCCAGCTTCTGGTACTCAATCAACCCCAGATATTGGTCCACATGTGCAA |
| AX-94619314 | TCTTCACTGCTGATGGATTCGAATCAGAAACCTCAATGCAGTTGAAGCCT[Y ]CGTGCAGAGTCGGAGTTAGTTGTTACTAGAACAACTGCAACAGTTTTGGA |
| AX-95224829 | AGTAGTCTCAAGAAAGAATGGCGCTGAATACTAGGAATGCAATCGTTGCC[S ]TTCTCCTCCTCGTCGTGATGGTCTCAACACCAGCGGTAACTGCCCAAGAG |
| AX-94659431 | TATGGACCGCTGGCCATCGGTATCAACGCCGCATACATGCAGACATACAT[G |

| | |
|---|---|
| | ]GCGGAGTGTCATGCCCATACATATGCGGCAGGCACCTCGACCACGGTGT |
| AX-95224463 | GTCCTTGTCTATCATTTGAAACATCTGAGTGTAGTTCTCGATGTCCTCCG[S<br><br>]CGGTAAATTCTTGGCAACGACCTTCATTAATCATTGGAAAATGATTCACG |
| AX-94418429 | ACAGTTAAGGATCATATAATTCAGACAAAACAAATTGTCCCAACGGGATC[R<br><br>]AGCGAGCCAGGTTGCCAGCTTCACACTAGCTTGTAGATCTGGATGGAGAA |
| AX-94426996 | GCTGAACAAGACATGCCTCTTGTGCCCTCAACCTCAAATCCTCAATGAAT[Y<br><br>]GGCGGGGTGCGCTACGAGGTTCAGGAGGGAGGGTAGGCCATGATCGATCT |

Figure 4

Figure 5

Europäisches Patentamt

European Patent Office

Office européen des brevets

# EUROPEAN SEARCH REPORT

Application Number

EP 21 18 2738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RUFO RUBÉN ET AL: "From landraces to improved cultivars: Assessment of genetic diversity and population structure of Mediterranean wheat using SNP markers", PLOS ONE, vol. 14, no. 7, 15 July 2019 (2019-07-15), page e0219867, XP055879821, DOI: 10.1371/journal.pone.0219867 * the whole document * | 1-7 | INV. A01H1/04 |
| X | VERA VENDRAMIN ET AL: "Genomic tools for durum wheat breeding: de novo assembly of Svevo transcriptome and SNP discovery in elite germplasm", BMC GENOMICS, BIOMED CENTRAL LTD, LONDON, UK, vol. 20, no. 1, 10 April 2019 (2019-04-10), pages 1-16, XP021271689, DOI: 10.1186/S12864-019-5645-X * the whole document * | 1-7 | |
| X | POZNIAK C J ET AL: "Potential for detection of marker-trait associations in durum wheat using unbalanced, historical phenotypic data", MOLECULAR BREEDING, KLUWER ACADEMIC PUBLISHERS, DO, vol. 30, no. 4, 5 May 2012 (2012-05-05), pages 1537-1550, XP035140213, ISSN: 1572-9788, DOI: 10.1007/S11032-012-9737-4 * the whole document * | 1-7 | TECHNICAL FIELDS SEARCHED (IPC)<br><br>A01H |

-/--

The present search report has been drawn up for all claims

| Place of search | Date of completion of the search | Examiner |
|---|---|---|
| Munich | 25 January 2022 | Keller, Yves |

EPO FORM 1503 03.82 (P04C01)

## EUROPEAN SEARCH REPORT

**Application Number**

EP 21 18 2738

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | LE GOUIS J ET AL: "Genome-wide association analysis to identify chromosomal regions determining components of earliness in wheat", THEORETICAL AND APPLIED GENETICS ; INTERNATIONAL JOURNAL OF PLANT BREEDING RESEARCH, SPRINGER, BERLIN, DE, vol. 124, no. 3, 8 November 2011 (2011-11-08), pages 597-611, XP035007056, ISSN: 1432-2242, DOI: 10.1007/S00122-011-1732-3 * the whole document * | | |
| T | LI FAJI ET AL: "Genome-wide linkage mapping of yield-related traits in three Chinese bread wheat populations using high-density SNP markers", THEORETICAL AND APPLIED GENETICS, SPRINGER BERLIN HEIDELBERG, BERLIN/HEIDELBERG, vol. 131, no. 9, 1 June 2018 (2018-06-01), pages 1903-1924, XP036567721, ISSN: 0040-5752, DOI: 10.1007/S00122-018-3122-6 [retrieved on 2018-06-01] * the whole document * | | TECHNICAL FIELDS SEARCHED (IPC) |

-/--

The present search report has been drawn up for all claims

| Place of search | Date of completion of the search | Examiner |
|---|---|---|
| Munich | 25 January 2022 | Keller, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

**EUROPEAN SEARCH REPORT**

Application Number

EP 21 18 2738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MORA FREDDY ET AL: "Genome-wide association mapping of agronomic traits and carbon isotope discrimination in a worldwide germplasm collection of spring wheat using SNP markers", MOLECULAR BREEDING, SPRINGER NETHERLANDS, DORDRECHT, vol. 35, no. 2, 4 February 2015 (2015-02-04), pages 1-12, XP035468859, ISSN: 1380-3743, DOI: 10.1007/S11032-015-0264-Y [retrieved on 2015-02-04] * the whole document * | | |

-----

TECHNICAL FIELDS
SEARCHED        (IPC)

The present search report has been drawn up for all claims

| Place of search | Date of completion of the search | Examiner |
|---|---|---|
| Munich | 25 January 2022 | Keller, Yves |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 105256044 A

- CN 107794308 A

**Non-patent literature cited in the description**

- **PASCUAL, L. ; RUIZ, M. ; LÓPEZ-FERNÁNDEZ, M. et al.** Genomic analysis of Spanish wheat landraces reveals their variability and potential for breeding. *BMC Genomics,* 2020, vol. 21, 122
- **SORIANO, J.M. ; SANSALONI, C. ; AMMAR, K. ; ROYO, C.** Labelling Selective Sweeps Used in Durum Wheat Breeding from a Diverse and Structured Panel of Landraces and Cultivars. *Biology,* 2021, vol. 10, 258
- **MÉRIDA-GARCÍA R ; LIU G ; HE S ; GONZALEZ-DUGO V ; DORADO G ; GÁLVEZ S et al.** Genetic dissection of agronomic and quality traits based on association mapping and genomic selection approaches in durum wheat grown in Southern Spain. *PLoS ONE,* 2019, vol. 14 (2), e0211718
- **MANGINI G ; GADALETA A ; COLASUONNO P ; MARCOTULI I ; SIGNORILE AM ; SIMEONE R et al.** Genetic dissection of the relationships between grain yield components by genome-wide association mapping in a collection of tetraploid wheats. *PLoS ONE,* 2018, vol. 13 (1), e0190162
- **JIN H ; WEN WE ; LIU JD ; ZHAI SN ; ZHANG Y ; YAN J ; LIU ZY ; XIA XC ; HE ZH.** Genomewide QTL mapping for wheat processing quality parameters in a Gaocheng 8901/ Zhoumai 16 recombinant inbred line population. *Front Plant Sci,* 2016, vol. 7, 1032
- **LIU JD ; HE ZH ; RASHEED A ; WEN WE ; YAN J ; ZHANG PZ ; WAN YX ; ZHANG Y ; XIE CJ ; XIA XC.** Genome-wide association mapping of black point reaction in common wheat (Triticum aestivum L.). *BMC Plant Biol,* 2017, vol. 17, 220

- **SUN CW ; ZHANG FY ; YAN XF ; ZHANG XF ; DONG ZD ; CUI DQ ; CHEN F.** Genomewide association study for 13 agronomic traits reveals distribution of superior alleles in bread wheat from the yellow and Huai Valley of China. *Plant Biotechnol J,* 2017, vol. 15, 953-69
- **VALLURU R ; REYNOLDS MP ; DAVIES WJ ; SUKUMARAN S.** Phenotypic and genomewide association analysis of spike ethylene in diverse wheat genotypes under heat stress. *New Phytol,* 2017, vol. 214, 271-83
- **LORENZ AJ ; HAMBLIN MT ; JANNINK JL.** Performance of single nucleotide polymorphisms versus haplotypes for genome-wide association analysis in barley. *PLoS One,* 2010, vol. 5, e14079
- **GARG, B. ; LATA, C. ; PRASAD, M.** A study of the role of gene TaMYB2 and an associated SNP in dehydration tolerance in common wheat. *Mol. Biol. Rep.,* 2012, vol. 39, 10865-10871
- **WANG, S.-X. ; ZHU, Y.-L. ; ZHANG, D.-X. ; SHAO, H. ; LIU, P. ; HU, J.-B. ; ZHANG, H. ; ZHANG, H.-P. ; CHANG, C. ; LU, J. et al.** Genome-wide association study for grain yield and related traits in elite wheat varieties and advanced lines using SNP markers. *PLoS ONE,* 2017, vol. 12, e0188662
- **HUANG, S. ; SUN, L. ; HU, X. ; WANG, Y. ; ZHANG, Y. ; NEVO, E. ; PENG, J. ; SUN, D.** Associations of canopy leaf traits with SNP markers in durum wheat (Triticum turgidum L. durum (Desf.). *PLoS ONE,* 2018, vol. 13, e0206226
- **GUERRA, F.P. ; YÁÑEZ, A. ; MATUS, I. ; DEL POZO, A.** Genome-Wide Association of Stem Carbohydrate Accumulation and Remobilization during Grain Growth in Bread Wheat (Triticum aestivum L.) in Mediterranean Environments. *Plants,* 2021, vol. 10, 539